# EUROPEAN PATENT APPLICATION

(11) **EP 4 574 145 A1**
(43) Date of publication of application: **25.06.2025**
(21) Application number: 23219377.1
(22) Date of filing: 21.12.2023
(51) Int. Cl.: A61K 31/198, A61K 31/047, A61K 31/201, A61K 31/202, A61K 31/401, A61K 31/7004, A61P 17/02

(54) **COMPOSITION COMPRISING AMINO ACIDS, METABOLIZABLE AND LIPID COMPONENT**

(71) Applicant: Paul Hartmann Aktiengesellschaft, 89522 Heidenheim (DE)
(72) Inventor: Smola, Hans, 89522 Heidenheim (DE); HERBIG, Michael, 22525 Hamburg (DE); KÖLLMER, Melanie, 22525 Hamburg (DE); GORISSEN, Sascha, 22525 Hamburg (DE)
(74) Representative: Ter Meer Steinmeister & Partner

(57) **Abstract**

The present invention relates to a composition, preferably for treating a wound, comprising (i) a proteinogenic amino acids component comprising 15 to 65 wt.% of glycine, 15 to 65°wt.% of proline, and 20 to 70 wt.% of accumulated branched proteinogenic amino acids based on the total weight of the proteinogenic amino acids component (i), (ii) a metabolizable component selected from glycerol and a carbohydrate selected from pentoses and hexoses, (iii) a lipid component, (iv) optionally excipients and (v) water. The invention further relates to the present composition for topical administration and to the present composition for use in wound treatment.

## Description

The present invention relates to a composition, preferably for treating a wound, comprising (i) a proteinogenic amino acid component comprising 15 to 65 wt.% of glycine, 15 to 65°wt.% of proline, and 20 to 70 wt.% of accumulated branched proteinogenic amino acids based on the total weight of the proteinogenic amino acids component (i), (ii) a metabolizable component selected from glycerol and a carbohydrate selected from pentoses and hexoses, (iii) a lipid component, (iv) optionally excipients and (v) water. The invention further relates to the present composition for topical administration and to the present composition for use in wound treatment.

### Technical Background

A wound can be regarded as separation of the contiguity of tissues of the skin, wherein this can be combined with a loss of substance.

The healing of wounds is based on the ability of the skin to regenerate tissue, such as epithelial tissue, connective and supporting tissue. The regeneration of tissue is a complex occurrence of cell activities overlapping each other, wherein said cell activities promote the healing process step by step. According to the literature, there are four main phases of healing. In the first phase, the hemostasis phase, the bleeding is stopped, and thrombin initiates the formation of a fibrin mesh. In the second phase, the defensive/inflammatory phase, specific white blood cells and macrophages successively enter the wound to destroy bacteria and remove debris. In addition, growth factors are provided to attract immune system cells to the wound and to facilitate tissue repair. The third phase is the proliferative phase. After the cleaning of the wound, said phase is to fill and cover the wound. During wound filling, granulation tissue fills the wound bed with connective tissue, and new blood vessels are formed. Subsequently, the wound margins contract and pull towards the wound center. In the last stage of said phase, epithelial cells are formed which cover the wound with epithelium. The fourth phase is the maturation phase. In said phase, the new tissue matures, i.e. it becomes stronger and more flexible with the reorganization of collagen fibers.

There are several approaches to support the distinct healing phases of a wound. For example, in WO 2010/000451 and WO 2011/141454 there are wound dressings ensuring a humid environment of the wound to promote the healing process. Further, there are attempts to promote the healing process by providing different components, such as amino acids, directly to the wound. For example, Maggio et al.: "A new protocol for the treatment of the chronic venous ulcers of the lower limb", (2012), Clin Exp Med 12, 55-60 describe a novel dressing in form of a powder containing glycine, L-lysine, L-proline, L-leucine and hyaluronic acid for the management of chronic ulcers, such as chronic venous ulcers of the lower limb. In this powder, the amino acids appear to be prone to oxidative phosphorylation such that the supporting effect to the wound healing might be negatively affected over time. US 11,737,998 B2 (Xie et al.) relates to a composition of a burn wound comprising a variety of ingredients. In particular, said composition comprises 261 to 639 parts of a fatty acid, in particular linoleic acid; 3.5 to 350 parts of an organic substance; 0.02 to 60 parts of a carbohydrate, in particular glucose; 0.009 to 0.013 parts of vitamin E; 3.04 to 5.5 parts of a microelement, in particular calcium; 0.6 to 70 parts of an antibiotic; and 105 to 335 parts of amino acid. This composition might be regarded as disadvantageous due to the high content of fatty acids. Further, US 8,404,661 B1 relates to pharmaceutical or cosmetic compositions in the form of a cream based on amino acids and sodium hyaluronate which can be used for wound healing or cosmetic purposes.

However, the compositions available at present still seem to be improvable with regard to their ingredients and amounts thereof. For example, for some patients use of hyaluronic acid as an essential component might be not desirable. Further, the types and amounts of proteinogenic acids used in the prior art are improvable.

Hence, it is an object of the present invention to provide a composition which overcomes the shortcomings of the prior art.

In particular, a composition should be provided enabling advantageous wound healing, especially with regard to the reduction of the open wound area and prevention of scarring.

Furthermore, advantageous healing properties should be achieved also for wounds having a reduced cell activity and/or cell density and at wounds of patients suffering from cachexia.

Moreover, a composition for treating poorly healing wounds, such as chronic and/or ischemic wounds, should be provided.

The above applies also to wounds at sites with no or strongly reduced arterial blood supply.

In particular, said composition should enable the progression of a chronic wound to the proliferative phase.

In summary, there is still a need for a composition for treating wounds, in particular poorly healing wounds.

Hence, it was an object of the present invention to overcome at least one of the above problems.

### Summary of the Invention

It has been unexpectedly found that the above objectives can be solved by the composition of the present invention.

Thus, the subject of the invention is a composition for treating a wound, comprising
(i) 1 to 5 wt. % of the proteinogenic amino acid component comprising
   - 15 to 65 wt.% of glycine,
   - 15 to 65°wt.% of proline and
   - 20 to 70 wt.% of accumulated branched proteinogenic amino acids, based on the total weight of the proteinogenic amino acids component (i),
(ii) 0.5 to 10 wt.% of a metabolizable component selected from glycerol and a carbohydrate component selected from pentoses and hexoses,
(iii) 5 to 15 wt.% of a lipid component,
(iv) optionally excipients and
(v) water to sum up to 100 wt.%
based on the total weight of the composition.

A further subject of the present invention is the use of the present composition in wound treatment, preferably for use in the treatment of ischemic and/or chronic wounds.

### Detailed description of the invention

In a first aspect, the present invention concerns a composition for treating a wound, wherein said composition comprises different components.

Component (i) is a proteinogenic amino acid component. The composition according to the present invention comprises the proteinogenic amino acid component (i) in a content of 1 to 5 wt.%, preferably 1.5 to 4.5 wt.%, more preferably 2.0 to 4.0 wt.%, even more preferably 2.5 to 3.5 wt.%, in particular about 3 wt.%, based on the total weight of the composition. Preferably, the present composition comprises proteinogenic amino acid component (i) in a content of about 2.0 wt.%, about 2.1 wt.%, about 2.2 wt.%, about 2.3 wt.%, about 2.4 wt.%, about 2.5 wt.%, about 2.6 wt.%, about 2.7 wt.%, about 2.8 wt.%, about 2.9 wt.%, about 3.0 wt.%, about 3.1 wt.%, about 3.2 wt.%, about 3.3 wt.%, about 3.4 wt.%, about 3.5 wt.%, about 3.6 wt.%, about 3.7 wt.%, about 3.8 wt.%, about 3.9 wt.% or about 4.0 wt.% based on the total weight of the composition.

Proteinogenic amino acids can be referred to as amino acids that are incorporated into proteins during translation via biosynthesis. In other words, the term "proteinogenic" means "protein creating". For humans, there are 21 proteinogenic amino acids, i.e. selenocysteine as well as and twenty (canonical) amino acids which are alanine (Ala), arginine (Arg), asparagine (Asn), aspartic acid (Asp), cysteine (Cys), glutamine (Gln), glutamic acid (Glu), glycine (Gly), histidine (His), isoleucine (Ile), leucine (Leu), lysine (Lys), methionine (Met), phenylalanine (Phe), proline (Pro), serine (Ser), threonine (Thr), tryptophane (trp), tyrosine (Tyr) and valine (Val). All of these amino acids are L-amino acids.

The proteinogenic amino acid component (i) comprises glycine in a content of 15 to 65 wt.%, preferably 16.5 to 55 wt.%, more preferably 17 to 45 wt.%, even more preferably 17.5 to 35 wt.%, in particular about 20 wt.%, based on the total weight of the proteinogenic amino acid component (i). Preferably, the proteinogenic amino acid component (i) comprises glycine in a content of about 15.5 wt.%, about 16 wt.%, about 16.5 wt.%, about 17 wt.%, about 17.5 wt.%, about 18 wt.%, about 18.5 wt.%, about 19 wt.%, about 19.5 wt.%, about 20 wt.%, about 20.5 wt.%, about 21 wt.%, about 21.5 wt.%, about 22 wt.%, about 22.5wt.%, about 23 wt.%, about 23.5 wt.%, about 24 wt.%, about 24.5 wt.% or about 25 wt.%, based on the total weight of the proteinogenic amino acids component (i).

The proteinogenic amino acids component (i) comprises proline in a content of 15 to 65 wt.%, preferably 16.5 to 55 wt.%, more preferably 17.5 to 45 wt.%, even more preferably 20 to 35 wt.%, in particular about 23 wt.%, based on the total weight of the proteinogenic amino acids component (i). Preferably, the proteinogenic amino acids component (i) comprises glycine in a content of about 18 wt.%, about 18.5 wt.%, about 19 wt.%, about 19.5 wt.%, about 20 wt.%, about 20.5 wt.%, about 21 wt.%, about 21.5 wt.%, about 22 wt.%, about 22.5wt.%, about 23 wt.%, about 23.5 wt.%, about 24 wt.%, about 24.5 wt.%, about 25 wt.%, about 25.5 wt.%, about 26 wt.%, about 26.5 wt.%, about 27 wt.%, about 27.5 wt.% or about 28 wt.%, based on the total weight of the proteinogenic amino acid component (i).

The proteinogenic amino acids component (i) comprises accumulated glycine and proline in a content of 35 to 70 wt.%, preferably 32.5 to 65 wt.%, more preferably 35 to 55 wt.%, even more preferably 40 to 50 wt.%, in particular about 44 wt.%, based on the total weight of the proteinogenic amino acids component (i).

Preferably, the proteinogenic amino acid component (i) comprises a glycine and proline in a content of about 35 wt.%, about 36 wt.%, about 37 wt.%, about 38 wt.%, about 39 wt.%, about 40 wt.%, about 41 wt.%, about 42 wt.%, about 43 wt.%, about 44 wt.%, about 45 wt.%, about 46 wt.%, about 47 wt.%, about 48 wt.%, about 49 wt.%, about 50 wt.%, about 51 wt.%, about 52 wt.%, about 53 wt.%, about 54 wt.% or about 55 wt.%, based on the total weight of the proteinogenic amino acids component (i).

The proteinogenic amino acids component (i) comprises accumulated branched proteinogenic amino acid(s) in a content of 20 to 70 wt.%, preferably 22.5 to 60 wt.%, more preferably 25 to 50 wt.%, even more preferably 30 to 40 wt.%, in particular about 33 wt.%, based on the total weight of the proteinogenic amino acids component (i). A branched proteinogenic amino acid is a proteinogenic amino acid having a branched alkyl residue. The proteinogenic amino acids component (i) can comprise one or more branched proteinogenic amino acids.

Preferably, the proteinogenic amino acids component (i) comprises accumulated branched proteinogenic amino acid(s) in a content of about 23 wt.%, about 24 wt.%, about 25 wt.%, about 26 wt.%, about 27 wt.%, about 28 wt.%, about 29 wt.%, about 30 wt.%, about 31 wt.%, about 32 wt.%, about 33 wt.%, about 34 wt.%, about 35 wt.%, about 36 wt.%, about 37 wt.%, about 38 wt.%, about 39 wt.%, about 40 wt.%, about 41 wt.%, about 42 wt.% or about 43 wt.%, based on the total weight of the proteinogenic amino acids component (i).

The branched proteinogenic acids in proteinogenic amino acids component (i) are preferably valine, leucin and isoleucine.

In a preferred embodiment, the proteinogenic amino acids component (i) comprises valine in a content of 7 to 25 wt.%, preferably 9 to 20 wt.%, more preferably 10 to 18 wt.%, even more preferably 11 to 16 wt.%, in particular about 13 wt.%, based on the total weight of the proteinogenic amino acids component (i). Preferably, proteinogenic amino acids component (i) comprises valine in a content of about 8 wt.%, about 8.5 wt.%, about 9 wt.%, about 9.5 wt.%, about 10 wt.%, about 10.5 wt.%, about 11 wt.%, about 11.5 wt.%, about 12 wt.%, about 12.5wt.%, about 13 wt.%, about 13.5 wt.%, about 14 wt.%, about 14.5 wt.%, about 15 wt.%, about 15.5 wt.%, about 16 wt.%, about 16.5 wt.%, about 17 wt.%, about 17.5 wt.% or about 18 wt.%, based on the total weight of the proteinogenic amino acids component (i).

In a preferred embodiment, the proteinogenic amino acids component (i) comprises leucine in a content of 10 to 25 wt.%, preferably 11 to 22 wt.%, more preferably 12 to 20 wt.%, even more preferably 14 to 19 wt.%, in particular about 16 wt.%, based on the total weight of the proteinogenic amino acids component (i). Preferably, the proteinogenic amino acids component (i) comprises leucine in a content of about 11 wt.%, about 11.5 wt.%, about 12 wt.%, about 12.5 wt.%, about 13 wt.%, about 13.5 wt.%, about 14 wt.%, about 14.5 wt.%, about 15 wt.%, about 15.5wt.%, about 16 wt.%, about 16.5 wt.%, about 17 wt.%, about 17.5 wt.%, about 18 wt.%, about 18.5 wt.%, about 19 wt.%, about 19.5 wt.%, about 20 wt.%, about 20.5 wt.% or about 21 wt.%, based on the total weight of the proteinogenic amino acids component (i).

In a preferred embodiment, the proteinogenic amino acids component (i) comprises isoleucine in a content of 1.5 to 10 wt.%, preferably 2 to 8 wt.%, more preferably 2.5 to 7 wt.%, even more preferably 3 to 5 wt.%, in particular about 4 wt.%, based on the total weight of the proteinogenic amino acids component (i). Preferably, the proteinogenic amino acids component (i) comprises isoleucine in a content of about 1.5 wt.%, about 2 wt.%, about 2.5 wt.%, about 3 wt.%, about 3.5 wt.%, about 4 wt.%, about 4.5 wt.%, about 5 wt.%, about 5.5 wt.%, about 6 wt.%, about 6.5 wt.%, about 7 wt.%, about 7.5 wt.%, or about 8 wt.%, based on the total weight of the proteinogenic amino acids component (i).

Component (ii) is a metabolizable component selected from glycerol and a carbohydrate component selected from pentoses and hexoses.

In line with the present application a metabolizable component refers to a component capable of being utilized in metabolism, preferably in metabolism of the human. It is preferred that, when being metabolized, component (ii) can provide energy which can for example be used in the energy-consuming processes during wound healing.

Pentoses can for example be aldopentoses, such as ribose, arabinose, xylose and lyxose, or ketopentoses, such as ribulose and xylulose. Hexoses can for example be aldohexoses, such as allose, altrose, glucose, mannose, gulose, idose, galactose and talose, or ketohexoses, such as psicose, fructose, sorbose and tagatose. Hexoses are preferred, in particular fructose and/or glucose. When used for a human being having a milk intolerance, it is preferable not to take galactose as hexose.

The composition according to the present invention comprises the metabolizable component (ii) selected from glycerol and a carbohydrate component selected from pentoses and hexoses in a content of 0.5 to 10 wt.%, preferably 1.5 to 8.5 wt.%, more preferably 2.0 to 7.5 wt.%, even more preferably 3.0 to 6.5 wt.%, in particular about 5wt.%, based on the total weight of the composition. Preferably, the present composition comprises said component (ii) in a content of about 2.0 wt.%, about 2.5 wt.%, about 3.0 wt.%, about 3.5 wt.%, about 4.0 wt.%, about 4.5 wt.%, about 5.0 wt.%, about 5.5 wt.%, about 6.0 wt.%, about 6.5 wt.%, about 7.0 wt.%, about 7.5 wt.%, or about 8.0 wt.%, based on the total weight of the composition.

Component (iii) is a lipid component. A lipid can be referred to as the entirety of fats or fat-like substances. Usually, a lipid is a class of organic compounds such as fatty acids or their derivatives that are poorly soluble or insoluble in water but soluble in organic (nonpolar) solvents ("lipophilia"). Lipids for example include many natural oils and waxes.

The composition according to the present invention comprises a lipid component (iii) in a content of 5 to 15 wt.%, preferably 6 to 14 wt.%, more preferably 7 to 13 wt.%, even more preferably 8 to 12 wt.%, in particular about 10 wt.%, based on the total weight of the composition. Preferably, the present composition comprises the lipid component (iii) in a content of about 7.5 wt.%, about 8.0 wt.%, about 8.5 wt.%, about 9.0 wt.%, about 9.5 wt.%, about 10.0 wt.%, about 10.5 wt.%, about 11.0 wt.%, about 11.5 wt.%, about 12.0 wt.% or about 12.5 wt.% based on the total weight of the composition.

In a preferred embodiment, the lipid component (iii) comprises fatty acids and/or triglycerides.

Fatty acids are mono carboxylic acids having, apart from the carbolic residue (-COOH), a saturated or unsaturated substantially non-branched hydrocarbon chain. Generally, fatty acids have an even number of carbon atoms. Fatty acids can be subdivided into lower fatty acids having up to seven carbon atoms, medium fatty acids having eight to twelve carbon atoms and higher fatty acids having more than twelve carbon atoms, preferably up to twenty-six carbon atoms.

Preferred are medium and higher fatty acids, more preferably higher fatty acids.

Examples of medium fatty acids are saturated fatty acids, such as caprylic acid (C₈H₁₆O₂), pelargonic acid (C₉H₁₈O₂), capric acid (C₁₀H₂₀O₂), capric acid (C₁₂H₂₄O₂) and unsaturated fatty acids such as undecylenic acid (C₁₀H₁₈O₂).

Examples of higher fatty acids are saturated fatty acids, such as myristic acid (C₁₄H₂₈O₂), palmitic acid (C₁₆H₃₂O₂), margaric acid (C₁₇H₃₄O₂), stearic acid (C₁₈H₃₆O₂), arachidic acid (C₂₀H₄₀O₂),behenic acid (C₂₂H₄₄O₂), lignoceric acid (C₂₄H₄₈O₂), cerotic acid (C₂₆H₅₂O₂) and unsaturated fatty acids such as palmitoleic acid (C₁₆H₃₀O₂), oleic acid (C₁₈H₃₄O₂), eicosenoic acid (C₂₀H₃₈O₂), cetoleic acid (C₂₂H₄₂O₂), erucic acid (C₂₂H₄₂O₂), nervonic acid (C₂₄H₄₆O₂), linoleic acid (C₁₈H₃₂O₂), arachidonic acid (C₂₀H₃₂O₂), eicosapentanoic acid (C₂₀H₃₀O₂) and docosahexaenoic acid (C₂₂H₃₂O₂).

Preferably the lipid component (iii) comprises an omega-9 fatty acid and/or an omega-3 fatty acid.

Omega-9 fatty acids are fatty acids wherein the final carbon-carbon double bound in a multiple unsaturated hydrocarbon chain is present in the omega-9 position, i.e. the ninth bond from the methyl end of the hydrocarbon chain. Examples are oleic acid, erucic acid and nervonic acid. Oleic acid is preferred.

Omega-3 fatty acids are fatty acids wherein the final carbon-carbon double bound in a multiple unsaturated hydrocarbon chain is present in the omega-3 position, i.e. the third bond from the methyl end of the hydrocarbon chain. Examples are linolenic acid, eicosapentaenoic acid (C₂₀H₃₀O₂) and docosahexaenoic acid (C₂₂H₃₂O₂).

Triglycerides are esters derived from glycerol and three fatty acids. These three fatty acids can be the same or they can be different. Further, it is possible that two of the fatty acids are the same and the third one is different. As far as fatty acids are concerned, the same generally applies as described above.

In a preferred embodiment of the present invention the lipid component (iii) comprises a triglyceride wherein at least one of the esterified acids is an esterified omega-9 fatty acid or an esterified omega-3 fatty acid. As far as omega-9 fatty acid or omega-3 fatty acid is concerned, also the same applies as described above.

It is preferred that the component (iii) comprises oleic acid, sweet almond oil or a mixture thereof. Sweet almond oil is a fatty plant oil obtained from the corresponding almonds via cold pressing.

In one preferred embodiment of the invention the present composition comprises one or more physiologically acceptable excipients. Physiologically acceptable excipients are known in the art. All such physiologically acceptable excipients must be substantially pharmaceutically pure and non-toxic in the amounts employed and must be compatible with the active ingredients.

In one preferred embodiment of the invention and/or embodiments thereof the physiologically acceptable excipients are selected from emulsifiers, antioxidants, gel-forming agents, stabilizers, preservatives, pH-adjusting agents, vitamins and mixtures thereof.

Emulsifiers are substances which combine two or more, preferably two, non-miscible fluids to a finely dispersed mixture, the so-called emulsion, and to stabilize said emulsion. Emulsifiers generally have a non-polar part, such as an alkyl group, and a polar part having a hydroxy group, a (poly) ethoxylate group, a carboxylic group, a sulfonate group, a sulfate group, and/or a quaternary amino group. Common emulsifiers are alkylsulfates, alkyltrimethylammonium salts, polyethylene glycol alkyl ethers, alcohol ethoxylates and the like. Preferred are polyethylene glycol alkyl ethers such as a polyethylene glycol octadecyl ether (C₁₈H₃₇(OCH₂CH₂)₂-OH. It is preferred that emulsifiers comprised in the present composition may be in the range of from 0.1 to 15 wt.%, more preferably from 1 to 13 wt.%, even more preferably from 3 to 11 wt.%, in particular about 9 wt.%, based on the total weight of the composition.

Antioxidants are substances that are used to inhibit oxidation. Antioxidants suitable to be comprised in the present composition include, but are not limited to, ascorbic acid, glutathione, tocopherol and its esters, tert-butylhydroquinone (TBHQ), butylated hydroxy anisole (BHA also referred to as 2-tert-butyl-4-hydroxy anisole), 3-tert-butyl-4-hydroxy anisole or a mixture thereof) and butyl hydroxy toluene (BHT also referred as 2,6-di-tert-butyl 4-methyl phenol). Preferred is butylated hydroxy anisole (BHA). It is preferred that antioxidants comprised in the composition may be in the range of from 0.001 to 1.00 wt.%, more preferably from 0.005 to 0.5 wt.%, in particular about 0.02 wt.%, based on the total weight of the composition.

Gel-forming agents can be referred to to as synthetic or natural polymeric substances, preferably hydrophilic synthetic or natural polymeric substances. These gel-forming synthetic or natural polymeric substances are capable of forming a gel, preferably a hydrogel, within a liquid, preferably water. The synthetic or natural polymeric substance can be regarded as a matrix.

The gel-forming agent comprised by the present composition can be preferably based on synthetic polymeric material selected from polyvinyl alcohol, polyalkylene oxide-based hydrogel, poly(meth)acrylate-based hydrogel, poly(eth)acrylate-based hydrogel, poly alkyl(meth)acrylate-based hydrogel, poly alkyl(meth)acrylate-based hydrogel, vinyl polymer-based hydrogel, polycaprolactam- and polycaprolactone-based hydrogel, polyurethane-based hydrogel, polyurea-based hydrogel and polyurethane-polyurea-copolymer-based hydrogel.

Alternatively preferably, the gel-forming agent comprised by the present composition can be preferably based on natural polymeric material selected from starch, dextran, agarose, pectin, alginate, chitosan, hyaluronic acid, gellan, polypeptide and cellulose. Optionally, the natural polymeric material can be further processed, for example by chemical derivation, such as the formation of esters and ethers or pharmaceutically acceptable salts.

It is particularly preferred that the gel-forming agent is cellulose and/or derivatives thereof. Cellulose is a polysaccharide containing a linear chain of several hundred to many thousands of β(1→4)-linked D-glucose units. Within the present application cellulose derivatives are for example cellulose ethers and cellulose esters as well as their salts. Examples of cellulose ethers are hydroxyalkyl cellulose, in particular hydroxy C₁₋₆-alkyl cellulose, such as hydroxymethyl cellulose, hydroxyethyl cellulose, hydroxypropyl cellulose, hydroxyisopropyl cellulose and hydroxybutyl cellulose, preferably hydroxymethyl cellulose and/or hydroxyethyl cellulose. Examples of cellulose esters are carboxyalkyl cellulose, in particular carboxy C₁₋₆-alkyl cellulose, such as carboxymethyl cellulose, carboxy ethyl cellulose, carboxypropyl cellulose, carboxybutyl cellulose and/or their salts. Further, a mixture of the mentioned compounds can be used. The number average molecular weight of cellulose and/or its derivates is 1 000 g/mol to 250 000 g/mol, preferably 5 000 g/mol to 175 000 g/mol, in particular 10 000 g/mol to 100 000 g/mol, determined with gel permeation chromatography.

Most preferred as gel-forming agent is hydroxyethyl cellulose. Hydroxyethyl cellulose can be obtained by reacting alkali cellulose with ethylene oxide.

It is preferred that gel-forming agents in the composition may be in the range of from 1 to 10 wt.%, more preferably from 2 to 7 wt.%, in particular about 4 wt.%, based on the total weight of the composition.

A stabilizer is a physiologically acceptable excipient which helps to preserve a product and/or to stabilize a gel. Examples include, but are not limited to, alginates, carrageen, gelatine, and EDTA-Na (ethylenediaminetetraacetic acid di sodium salt), preferably EDTA-Na (ethylenediaminetetraacetic acid disodium salt. It is preferred that stabilizers in the composition may be in the range of from 0.001 to 0.1 wt.%, more preferably from 0.005 to 0.05 wt.%, in particular about 0.01 wt.%, based on the total weight of the composition.

Preservatives are substances that can be added to prevent decomposition by microbial growth or by undesirable chemical changes. Non-limiting examples include lactic acid, benzoic acid, benzoates, hydroxybenzoates and benzyl alcohol. Preferred is benzyl alcohol. It is preferred that preservatives in the composition may be in the range of from 1 to 5 wt.%, more preferably from 1.5 to 3 wt.%, in particular about 2 wt.%, based on the total weight of the composition.
pH-adjusting agents are substances to maintain/adjust the pH value of a product. The pH value of the composition is between 4 and 8.5, preferably between 4.5 and 5.5. Non-limiting examples of pH-adjusting agents are hydrochloric acid (1 mol/L) if the pH of the composition should be decreased, and sodium hydroxide solution in water (1 mol/L) if the pH of the composition should be raised. It is preferred that pH-adjusting agents in the composition may be in the range of from 0.05 to 0.5 wt.%, more preferably from 0.1 to 0.3 wt.%, in particular about 0.2 wt.%, based on the total weight of the composition.

Further, the composition may comprise vitamins. Vitamins are essential to an organism in small quantities for proper metabolic function. The vitamins include vitamin A, vitamin B1, vitamin B2, vitamin B3, vitamin B5, vitamin B6, vitamin B7, vitamin B9, vitamin B12, vitamin C, vitamin D, vitamin E, vitamin K and choline.

It is preferred that accumulated vitamins in the composition may be in the range of from 0.00001 to 10 wt.%, preferably 0.00005 to 5wt.%, in particular 0.0001 to 1 wt.% based on the total weight of the composition.

Most preferred, the composition comprises 7 µg to 900 µg Vitamin A, 11 µg to 1200 µg Vitamin B1, 11 µg to1300 µg Vitamin B2, 140 µg to 1600 µg Vitamin B3, 50 µg to 5000 µg Vitamin B5, 13 µg to 1300 µg Vitamin B6, 0.3 µg to 30 µg Vitamin B7, 4 µg to 400 µg Vitamin B9, 0.02 µg to 2.4 µg Vitamin B12, 750 µg to 90000 µg Vitamin C, 0.15 µg to15 µg Vitamin D, 150 µg to 15000 µg Vitamin E, and/or 0.9 µg to120 µg Vitamin K, each per gram of the composition.

Preferably the composition does not comprise the amino acid L-phenylalanine. A composition being devoid of phenylalanine can advantageously be used for treating wounds of patients suffering under the metabolic disease phenylketonuria.Further, it is preferred that the composition according to the present invention does not comprise hyaluronic acid and the pharmaceutically acceptable salts thereof.

Further, the composition preferably does not comprise ornithine and the pharmaceutically acceptable salts thereof.

Further, the present composition comprises water (v) to sum up to 100 wt.%.

In a preferred embodiment, the present composition comprises
(i) 1 to 5 wt.%, preferably 2.0 to 4.0 wt.%, more preferably 2.5 to 3.5 wt.% in particular about 3 wt.% of the proteinogenic amino acids component comprising
   - 15 to 65 wt.%, more preferably 17 to 45 wt.%, even more preferably 17.5 to 35 wt.%, in particular about 20 wt% of glycine,
   - 15 to 65°wt.%, preferably 17.5 to 45 wt.%, more preferably 20 to 35 wt.%, in particular about 23 wt.% of proline, and
   - 20 to 70 wt.%, preferably 25 to 50 wt.%, more preferably 30 to 40 wt.%, in particular about 33 wt.% of accumulated branched proteinogenic amino acids,
   - based on the total weight of the proteinogenic amino acids component (i),
(ii) 0.5 to 10 wt.%, preferably 2.0 to 7.5 wt.%, more preferably 3.0 to 6.5 wt.%, in particular about 5wt.% of a metabolizable component selected from glycerol and a carbohydrate component selected from pentoses and hexoses,
(iii) 5 to 15 wt.%, preferably 7 to 13 wt.%, more preferably 8 to 12 wt.%, in particular about 10wt.% of the lipid component
(iv)
   a) 0.1 to 15 wt.%, more preferably from 1 to 13, even more preferably from 3 to 11, in particular about 9 wt.% of emulsifier,
   b) 0.001 to 1.00 wt.%, more preferably from 0.005 to 0.5 wt.%, in particular about 0.02 wt.% of antioxidants,
   c) 1 to 10 wt.%, more preferably from 2 to 7 wt.%, in particular about 4 wt.% of gel-forming agent,
   d) 0.001 to 0.1 wt.%, more preferably from 0.005 to 0.05 wt.%, in particular about 0.01 wt.% of stabilizer,
   e) 1 to 5 wt.%, more preferably from 1.5 to 3 wt.%, in particular about 2 wt.% of preservative,
   f) 0.05 to 0.5 wt.%, more preferably from 0.1 to 0.3 wt.%, in particular about 0.2 wt.% of pH-adjusting agent,
   g) 0.00001 to 10 wt.%, preferably 0.00005 to 5wt.%, in particular 0.0001 to 1 wt.% of accumulated vitamins, and
(v) water to sum up to 100 wt.%
based on the total weight of the composition.

In a preferred embodiment, the present composition comprises
(i) about 3 wt.% of the proteinogenic amino acids component comprising
   - about 20 wt.% of glycine,
   - about 23 wt.% of proline, and
   - about 33 wt.% of accumulated branched proteinogenic amino acids,
   - based on the total weight of the proteinogenic amino acids component (i),
(ii) about 5 wt.% of a metabolizable component selected from glycerol and a carbohydrate component selected from pentoses and hexoses,
(iii) about 10wt.% of the lipid component
(iv)
   a) about 9 wt.% of emulsifier,
   b) about 0.02 wt.% of antioxidants,
   c) about 4 wt.% of gel-forming agent,
   d) about 0.01 wt.% of stabilizer,
   e) about 2 wt.% of preservative,
   f) about 0.2 wt.% of pH-adjusting agent,
   g) 0.0001 to 1 wt.% of accumulated vitamins, and
(v) water to sum up to 100 wt.%
based on the total weight of the composition.

In a preferred embodiment, the present composition comprises
(i) 1 to 5 wt.%, preferably 2.0 to 4.0 wt.%, more preferably 2.5 to 3.5 wt.% in particular about 3 wt.% of the proteinogenic amino acids component comprising
   - 15 to 65 wt.%, more preferably 17 to 45 wt.%, even more preferably 17.5 to 35 wt.%, in particular about 20 wt% of glycine,
   - 15 to 65°wt.%, preferably 17.5 to 45 wt.%, more preferably 20 to 35 wt.%, in particular about 23 wt.% of proline,
   - 9 to 20 wt.%, preferably 10 to 18 wt.%, more preferably 11 to 16 wt.%, in particular about 13 wt.% of valine,
   - 11 to 22 wt.%, more preferably 12 to 20 wt.%, even more preferably 14 to 19 wt.%, in particular about 16 wt.% of leucine, and
   - preferably 2 to 8 wt.%, more preferably 2.5 to 7 wt.%, even more
      preferably 3 to 5 wt.%, in particular about 4 wt.% of isoleucine, based on the total weight of the proteinogenic amino acids component (i),
(ii) 0.5 to 10 wt.%, preferably 2.0 to 7.5 wt.%, more preferably 3.0 to 6.5 wt.%, in particular about 5wt.% of glycerol,
(iii) 5 to 15 wt.%, preferably 7 to 13 wt.%, more preferably 8 to 12 wt.%, in particular about 10wt.% of accumulated oleic acid and sweet almond oil
(iv)
   a) 0.1 to 15 wt.%, more preferably from 1 to 13 wt.%, even more preferably from 3 to 11 wt.%, in particular about 9 wt.% of polyethylene glycol alkyl ethers,
   b) 0.001 to 1.00 wt. %, more preferably from 0.005 to 0.5 wt.%, in particular about 0.02 wt.% of butylated hydroxyanisole (BHA),
   c) 1 to 10 wt.%, more preferably from 2 to 7 wt.%, in particular about 4 wt.% of hydroxyethyl cellulose,
   d) 0.001 to 0.1 wt.%, more preferably from 0.005 to 0.05 wt.%, in particular about 0.01 wt.% of EDTA-Na,
   e) 1 to 5 wt.%, more preferably from 1.5 to 3 wt.%, in particular about 2 wt.%, of benzyl alcohol,
   f) 0.05 to 0.5 wt.%, more preferably from 0.1 to 0.3 wt.%, in particular about 0.2 wt.% of aqueous HCl (1 mol/L),
   g) 0.00001 to 10 wt.%, preferably 0.00005 to 5wt.%, in particular 0.0001 to 1 wt.% of accumulated vitamins, and
(v) water to sum up to 100 wt.%
based on the total weight of the composition.

In a preferred embodiment, the present composition comprises
(i) about 3 wt.% of the proteinogenic amino acids component comprising
   - about 20 wt% of glycine,
   - about 23 wt.% of proline,
   - about 13 wt.% of valine,
   - about 16 wt.% of leucine, and
   - about 4 wt.% of isoleucine,
   based on the total weight of the proteinogenic amino acids component (i),
(ii) about 5 wt.% of glycerol,
(iii) about 10 wt.% of accumulated oleic acid and sweet almond oil
(iv)
   a) about 9 wt.% of polyethylene glycol alkyl ethers,
   b) about 0.02 wt.% of butylated hydroxyanisole (BHA),
   c) about 4 wt.% of hydroxyethyl cellulose,
   d) about 0.01 wt.% of EDTA-Na,
   e) about 2 wt.%, of benzyl alcohol,
   f) about 0.2 wt.% of aqueous HCl (1 mol/L),
   g) 0.0001 to 1 wt.% of accumulated vitamins, and
(v) water to sum up to 100 wt.%
based on the total weight of the composition.

In a preferred embodiment, the composition according to the present invention is in form of an ointment or a gel. An ointment is a soft and spreadable homogenous preparation which is intended to be administered to skin or mucous membranes. A gel is a disperse system in which a fluid component is immobilized in a solid component which is usually soft and easily applicable to skin or membranes.

In a preferred embodiment the composition according to the present invention is for topical administration. Contrary to systemic administration, wherein a composition is administered systemically for example via a tablet or an infusion and thus remote from the site where the therapeutic effect should be achieved, the present composition is preferably administered topically, i.e. it is administered directly to the site, preferably a wound, where the therapeutic effect should take place.

In a further aspect, the present invention concerns compositions according to the present invention for use in wound treatment. The present composition can be used for the treatment of all kinds of wounds. Examples of wounds include, but art not limited to, acute wounds, such as cuts, chronic wounds, such as decubitus, diabetic foot lesions, venous leg ulcers, arterial leg ulcers and pressure ulcers, purulent wounds, such as closed wounds resulting from an operation, infected wounds due to a local infection of the wound, traumatic wounds, such as wounds resulting from mechanical, thermic, chemical or actinic impact and ischemic wounds which can occur due to poor blood flow.

It is preferred that the composition according to the present invention is used in the treatment of ischemic and/or chronic wounds.

As described above, ischemic wounds refer to wounds with a poor blood supply. Due to the poor blood supply the wound is not provided with all components necessary for a good wound healing. There are parts of the body which due to poor blood flow are prone to ischemic wounds, such as feet and the lower parts of the legs. However, an ischemic wound can also occur in a part of the body usually well supplied with blood when said blood supply is diminished in line with the occurrence of the wound, for example by a cut through or a blockage of the blood-leading vessels.

A wound is generally referred to as a chronic wound when the healing process cannot be finished after six to eight weeks. Chronic wounds are often due to a chronic disease and/or a chronic mechanical impact, such as friction which, for example, applies in case of decubitus.

In a preferred embodiment of the present invention, the ischemic and/or chronic wound is selected from diabetic foot lesions, venous leg ulcers, arterial leg ulcers and pressure ulcers.

In a preferred embodiment of the present invention, the present composition is administered topically in an amount of 0.05 to 3.18 g/cm² of the wound area. The composition can preferably be administered directly to the wound. In that case is it preferred that the composition is administered in an amount of 0.05 to 0.5 g/cm² of the wound area, more preferably in amount of about 0.1 g/cm² of the wound area. The composition can be administered from four times a day to every three days. It is further preferred that the composition is administered once daily.

In a preferred embodiment of the present invention, the present composition is administered topically and by using a receptable, which creates a reservoir. Using such a reservoir facilitates the application of high amounts of the composition, for example 3.18 g/cm² of the wound area, without the applied composition being displaced to the side when external pressure is applied. It is possible to use receptables such as the "platform wound device" as disclosed by Nuutila et al.: "Immediate Treatment of Brum Wounds with High Concentrations of Topical Antibiotics in an Alginate Hydrogel Using a Platform Wound Device", February 2020, Adv Wound Care (New Rochelle) 1;9(2): 48-60.

Generally, the composition according to the present invention can be administered at any phase of the wound healing. In a preferred embodiment of the present invention, the composition is administered during the inflammatory and/or the proliferative phase of the wound.

In a preferred embodiment of the present invention, the composition promotes the progress from a chronic inflammatory to the proliferative phase of the wound.

### Experimental Part

### 1. Preparation of a stock solution comprising proteinogenic amino acids

One litre of an aqueous proteinogenic amino acids-containing stock solution was prepared, wherein said solution contains about 6% proteinogenic amino acid.

For this purpose, the following amounts of the corresponding aqueous proteinogenic amino acids as listed in below Table A were dissolved in water.

**Table A:**

| **L-Amino acid** | **mg/L** | **Content based on the total amount of proteinogenic amino acids [%]** |
|---|---|---|
| Alanine | 890 | 1.5 |
| Arginine hydrochloride | 836.2 | 1.4 |
| Asparagine-H₂O | 369.4 | 0.6 |
| Aspartic acid | 24 | 0.0 |
| Cysteine hydrochloride-H₂O | 388.0 | 0.6 |
| Glutamic acid | 211.8 | 0.3 |
| Glutamine | 5138.2 | 8.5 |
| Glycine | 12431.68 | 20.5 |
| Histidine hydrochloride-H₂O | 763.4 | 1.3 |
| Isoleucine | 2439.8 | 4.0 |
| Leucine | 9680.4 | 16.0 |
| Lysine hydrochloride | 1649.0 | 2.7 |
| Methionine | 223.8 | 0.4 |
| Phenylalanine | 565.0 | 0.9 |
| Proline | 13926.2 | 23 |
| Serine | 718.8 | 1.2 |
| Threonine | 1000.6 | 1.7 |
| Tryptophan | 539.2 | 0.9 |
| Tyrosine disodium salt-2H₂O | 641.4 | 1.1 |
| Valine | 8188.8 | 13.0 |
| Sum | 60615.4 | 100 |

### 2. Preparation of a composition according to the invention

A composition in accordance with the present invention was prepared by mixing and stirring the components as listed in Table B.

**Table B: Components and their contents of the present composition**

| **Component** | **wt.%** |
|---|---|
| 6% amino stock solution of Example 1 | 50 |
| Benzyl alcohol ((USP-NF) | 2 |
| Butylated hydroxyanisole (BHA) | 0.02 |
| EDTA-Na (Titriplex) | 0.01 |
| Glycerol | 5 |
| HCl (1mol/L) | 0.2 |
| Hydroxyethyl cellulose (Natrosol 250 H PHARM) | 4 |
| Oleic acid (Cremer Ac Vegetable Oleic Acid | 2 |
| Polyethylene glycol alkyl ether (SP Brij S2 MBAL) | 1 |
| Polyethylene glycol alkyl ether (SP Brij S721 MBAL-PA-SG) | 1 |
| Sweet Almond Oil (USP29-NF24) | 8 |
| Water, deionized | 27.77 |

### 3. Preparation of a Reference composition

A Reference composition was prepared by mixing and stirring the components as listed in Table C.

**Table C: Components and their contents of a Reference composition**

| **Component** | **wt.%** |
|---|---|
| Benzyl alcohol ((USP-NF) | 2 |
| Butylated hydroxyanisole (BHA) | 0.02 |
| EDTA-Na (Titriplex) | 0.01 |
| HCl (1mol/L) | 0.1 |
| Hydroxyethyl cellulose (Natrosol 250 H PHARM) | 4 |
| Mixture of paraffins (White oil; Marcol 52 from Exxon Mobil) | 10 |
| Phosphate Buffer pH 4.0 (RaDes GmbH) | 81.87 |
| Polyethylene glycol alkyl ether (SP Brij S2 MBAL) | 1 |
| Polyethylene glycol alkyl ether (SP Brij S721 MBAL-PA-SG) | 1 |

### 4. Comparison of composition according to Example 2 and Reference composition according to Example 3 in an ischemic wound healing model in pigs

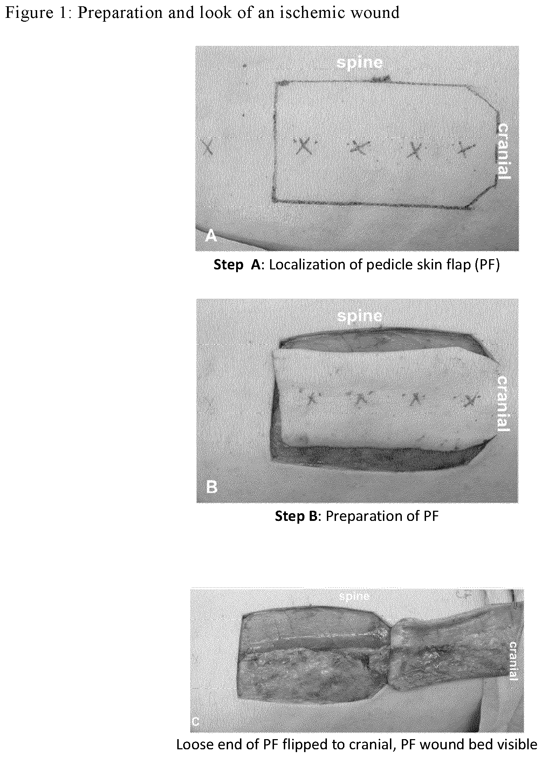

To model an ischemic wound healing, two "ischemic" wounds in the backs of pigs were generated according to the following surgical procedure, which is illustrated in the first two pictures in Figure 1:
Step A: Localization of the pedicle skin flap (PF)
Step B: Preparation of the pedicle skin flap (PF)

In the third picture it is shown that the loose end of the pedicle skin flap (PF) is flipped to the cranial direction and the wound bed is visible.

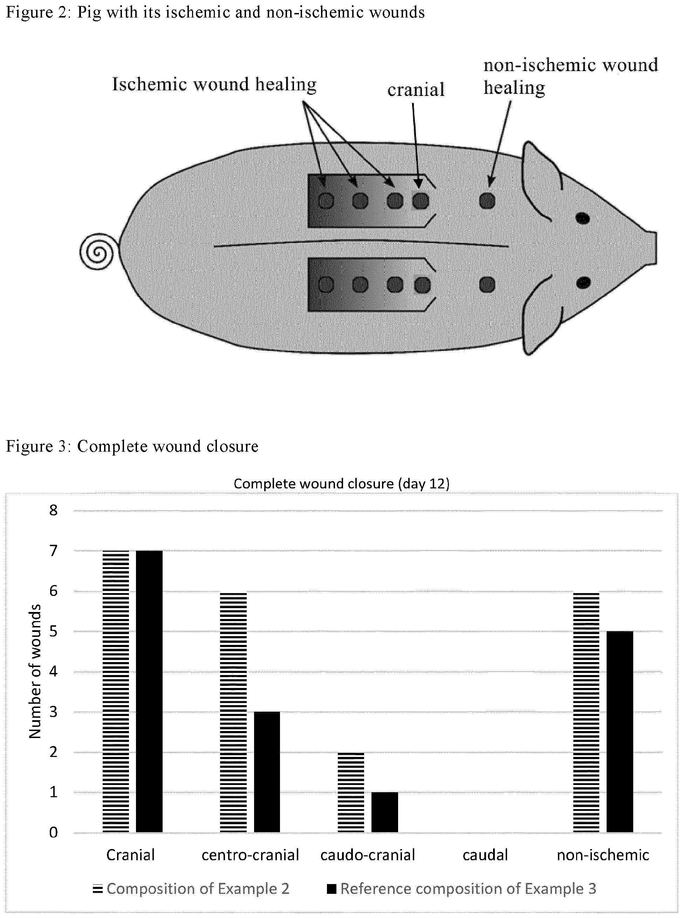

Figure 2 schematically shows a pig with its ischemic and two non-ischemic wounds.

**Procedure:** To one non-ischemic wound and one pedicle skin flap (PF) wound of one side of a pig, the present composition according to Example 2 was administered. To one non-ischemic wound and one pedicle skin flap (PF) wound of the other side of a pig, Reference composition according to Example 3 was administered. Subsequently, the number of complete closed wounds after 12 days as well as the wound area reduction after 7 and 12 days were determined by visual measurement and planimetry at the marked sites of the pedicle skin flap (PF) wounds and the non-ischemic wounds.

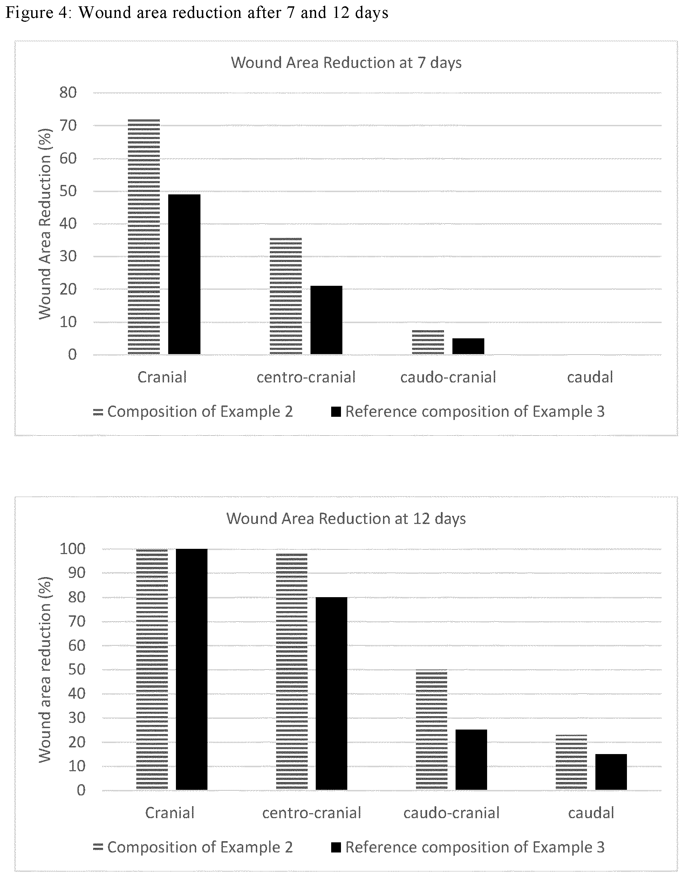

**Results:** The results are shown in Figures 3 and 4.

As can be seen from Figure 3, non-ischemic wound healing was only slightly improved with the present composition according to Example 2. Further, irrespective of whether the composition according to Example 2 or Reference composition according to Example 3 was administered, the healing of the caudal part of the pedicle skin flap (PF) wound was good. It is assumed that this is due to the good blood supply in the non-ischemic wound and the apparently still sufficient blood supply in the caudal part of the pedicle skin flap (PF) wound which is attached to tissue normally supplied with blood.

However, at the centro-cranial and caudo-central part, the number of closed wounds is double in case of the administration of the present composition according to Example 2 compared to the reference Composition. Thus, a significantly better wound healing can be achieved in these parts of the ischemic wound.

At the caudal end of the pedicle skin flap (PF) wound, none of the administered compositions achieved complete closure of said part of the wound. It is assumed that irrespective of the components directly administered to the wound, this part is too far away from a sufficient blood supply necessary for a complete healing of a wound.

Similar conclusions can be derived from Figure 4.

The wound area reduction at the cranial end of the pedicle skin flap (PF) wound is slightly better after 7 days, when the present composition according to Example 2 is used. After 12 days, both administered compositions achieve the same wound area reduction.

At the centro-cranial and caudo-central part, however, the wound area reduction is significantly higher when the present composition according to Example 2 is used.

At the caudal end of the pedicle skin flap (PF) wound, substantially no wound area reduction after 7 days can be achieved by any one of the administered compositions, while after 12 days the present composition according to Example 2 at least shows a wound area reduction of more than 20%.

## Claims

1. Composition for treating a wound, comprising
(i) 1 to 5 wt.% of the proteinogenic amino acids component comprising
- 15 to 65 wt.% of glycine,
- 15 to 65°wt.% of proline, and
- 20 to 70 wt.% of accumulated branched proteinogenic amino acids, based on the total weight of the proteinogenic amino acids component (i),
(ii) 0.5 to 10 wt.% of a metabolizable component selected from glycerol and a carbohydrate component selected from pentoses and hexoses,
(iii) 5 to 15 wt.% of the lipid component,
(iv) optionally excipients, and
(v) water to sum up to 100 wt.%,
based on the total weight of the composition.

2. Composition according to claim 1, wherein in the proteinogenic amino acids component (i) the accumulated content of glycine and proline is 35 to 70 wt.% based on the total weight of proteinogenic amino acids component (i).

3. Composition according to claim 1 or 2, wherein in the proteinogenic amino acids component (i) the accumulated content of the branched proteinogenic amino acids is 25 to 65wt.% based on the total weight of proteinogenic amino acids component (i).

4. Composition according to any one of claims 1 to 3, wherein the branched proteinogenic acids are valine, leucin and isoleucine and wherein the content of valine is 8 to 25 wt.%, the content of leucin is 10 to 25 wt.% and/or the content of isoleucine is 1.5 to 10wt.%, based on the total weight of the proteinogenic amino acids component (i).

5. Composition according to any one of clams 1 to 4, wherein the lipid component (iii) comprises fatty acids and/or triglycerides.

6. Composition according to any one of clams 1 to 5, wherein the lipid component (iii) comprises an omega-9 fatty acid and/or an omega-3 fatty acid.

7. Composition according to any one of clams 1 to 6, wherein the lipid component (iii) comprises a triglyceride, wherein at least one of the esterified acids is an esterified omega-9 fatty acid or an esterified omega-3 fatty acid.

8. Composition according to any one of clams 1 to 7, wherein the excipients comprise one or more excipients selected from emulsifiers, antioxidants, gel-forming agents, stabilizers, preservatives, pH-adjusting agents, vitamins and mixtures thereof.

9. Composition according to any one of clams 1 to 8 being in form of an ointment or a gel.

10. Composition according to any one of claims 1 to 9 for topical administration.

11. The composition according to any one of claims 1 to 10 for use in wound treatment, preferably for use in the treatment of ischemic and/or chronic wounds.

12. The composition for use according to claim 11, wherein the ischemic and/or chronic wound is selected from diabetic foot lesions, venous leg ulcers, arterial leg ulcers and pressure ulcers.

13. The composition for use according to any one of claims 11 or 12, wherein the composition is topically administered in an amount of 0.05 to 3.18 g/cm² of the wound area.

14. The composition for use according to any one of claims 10 to 12, wherein the composition is administered during the inflammatory and/or the proliferative phase of the wound.

15. The composition for use according to any one of claims 10 to 12, wherein the composition promotes the progress from a chronic inflammatory to the proliferative phase of the wound.
